# EUROPEAN PATENT APPLICATION

(11) **EP 0 621 046 A1**
(43) Date of publication of application: **26.10.1994**
(21) Application number: 94102383.0
(22) Date of filing: 17.02.1994
(51) Int. Cl.: A61M 1/14

(54) **Method of measuring the effect of a dialysis treatment**

(30) Priority: 05.03.1993 SE 9300728
(71) Applicant: GAMBRO AB, S-220 10 Lund (SE)
(72) Inventor: Sternby, Jan, S-222 52 Lund (SE)
(74) Representative: Asketorp, Göran

(57) **Abstract**

Method of measuring the effect of a dialysis or similar treatment whereby a fraction of the used dialysis fluid is extracted downstream of the dialyzer for analysis of at least one substance, i.e. urea, whereby said fraction of dialysis fluid is extracted via a branch conduit (8) containing a pump (6).

The method according to the invention is characterized in that the total amount of used dialysis fluid is measured, in that the proportion and/or amount of said substance in the extracted fraction is measured and also in that the total amount of said substance removed from the patient is calculated with the help of these values.

The invention is preferably intended for use in connection with haemodialysis, haemofiltration and haemodiafiltration.

## Description

### BACKGROUND TO THE INVENTION

The present invention relates of a method of measuring the effect of a dialysis or similar treatment, whereby a fraction of the used dialysis fluid is extracted downstream of the dialyzer for analysis of at least one substance, e.g. urea, whereby said fraction of said dialysis fluid is extracted via a branch conduit containing a pump.

The invention is primarily intended for use in connection with haemodialysis, but can also be used for instance in haemofiltration and haemodiafiltration which can be said to be something between haemodialysis and haemofiltration i.e. a dialysis treatment with the help of a dialyzer having a permeability such that the patient needs to be supplied with a replacement fluid for the lost plasma water.

### STATE OF THE ART

It has become increasingly important and more usual to use some form of quantifying the dialysis which is administered to a patient. The most common way is by use of the method which goes under the name of "Urea Kinetic Modelling (UKM)". This method is based on a measurement of the level of urea in the blood before and after each treatment. These values are put into a theoretical model which describes how the level of urea is changed in the blood during dialysis. In this model it is assumed that the blood's degree of purity in the dialyzer is given by clearance K (including the remaining function of the kidneys) and that this leads to a similarly large concentration c of urea in the whole of its distribution volume V in the body. If one neglects the production of urea in the body and the change in fluid volume during dialysis, one arrives at a treatment time T of

C_{after} = C_{before} * e^{-KT/V}

The coefficient KT/V is normally used as a measure of the administered dose of dialysis and can, in the above model, be calculated from the concentrations of urea before and after dialysis. This model can be corrected for the production of urea by taking a measurement of the concentration at the start of the next dialysis and can then also give a measure of the urea production which is indirectly a measure of the patient protein intake.

The assumptions which underlie the above model do not however always correspond with the reality of practical dialysis and result of the calculations performed is therefore not particularly reliable. Particular uncertainty occurs with high efficiency treatments, i.e. when using highly permeable membranes. This has thus produced a great debate concerning how one should measure and how one should calculate and use the results respectively.

Another and better alternative method is to measure the amount of removed urea in the used dialysis fluid. This can be done by collecting the used dialysis fluid and measuring both the concentration of urea and the total amount of used dialysis fluid. In this way an exact value of the removed amount of urea can be obtained, this being without the use of any theoretical model. The amount of urea removed in comparison to the weight of the body gives a correct value of the effect which is obtained through dialysis. By periodic measurement of the urea in the blood it can be ensured that the amount of urea in the body in the long run is unchanged. In this way the amount of urea removed also becomes a measure of the amount of urea produced, i.e. of the patient's protein intake.

To collect the complete amount of used dialysis fluid is however impractical. Different methods have thus been suggested for collecting only a fraction of the total amount of used dialysis fluid. According to one suggested method the used dialysis fluid should pass through a container with twenty-five identical holes. The fluid is retained in one of the twenty-five holes whilst the rest of the fluid is lead away to a drain. A problem with this method is that it is very difficult to make and maintain twenty-five holes with exactly the same size, which is necessary in order to obtain a correct determination of the total volume. The collected volume is moreover far to large to be practical. During a treatment of five hours this can be up to six litres. It is also not possible to increase the number of holes in order to reduce this volume. This would pose additional difficulties in determining the total volume.
In the article "Urea kinetic modelling by partial dialysate collection", in The International Journal of Artificial Organs/Vol 12/no 2, 1989/pp 96-102 by L J Garred, M Rittau, W McCready and B Canaud, there is described a process for calculation of the amount of urea removed from the patient whereby a sample is extracted downstream of the dialyzer. This sample is then analysed, after which the total amount of released urea is calculated by multiplying the concentration in this sample by the total dialysed amount from the dialyzer which is obviously determined by an estimation. Such an estimation is however difficult to perform since the amount of dialysis fluid pumped into the dialyzer does not have to correspond with the entered value. Furthermore in using this method one has to either measure or estimate the ultrafiltration in the dialyzer. In the article it is said that one makes use of "timed collection" several times in order to arrive at an average value. This requires a constant total flow, i.e. also a constant ultrafiltration, something which is difficult to achieve especially with haemofiltration and haemodiafiltration.

### SUMMARY OF THE INVENTION

Likewise according to the invention there is proposed a method to measure the effect of a dialysis treatment whereby a fraction of the used dialysis fluid is extracted downstream of the dialyzer for analysis of at least one substance, e.g. urea, whereby said fraction of dialysis fluid is extracted via a branch conduit containing a pump.

This new method is characterized in that the total amount of used dialysis fluid is measured, in that the portion and/or amount of said substance in the extracted fraction is measured and also in that the total amount of said substance removed from the patient is calculated with the help of these values. In this way there is no need to make any estimate of the total amount of used dialysis fluid but use can be made of a measured value which can be obtained with great exactitude e.g. by continually measuring the total flow from the dialyzer and integrating this flow with time.

Preferably the pump is made to operate with a speed which gives a flow proportional to the flow in the main conduit upstream of the branching point and downstream of the dialyzer. In this way it is guaranteed that the concentration in the extracted fraction will be the same as in the total dialysis amount.

When the pump is operated continuously, particularly accurate measured values are obtained. As is clear from the following, it can also be operated intermittently of course.

In practice it is possible to choose the flow in the branch conduit to be equal to one twentieth to one twenty-thousandth of the flow upstream of the branching point and downstream of the dialyzer. The flow is however conveniently chosen to lie between one two-hundredth and one ten-thousandth and preferably c:a one five-thousandth of said flow.

The proportion of many different substances can be measured in the extracted sample. However preferably the proportions of urea, creatine, phosphate or beta-micro-globulin are measured as well as a measure of the effect of the dialysis performed.

The sampling pump is conveniently started automatically or manually, when it can be established that the first blood is approaching or has reached the dialyzer. At the same time one should start the measuring of the total used amount of dialysis fluid inclusive of the extracted ultrafiltrate.

As mentioned above, the pump can also be operated intermittently. The number of sample extraction accuracies should thereby be selected to be at least ten, conveniently over fifty and preferably considerably higher, e.g. over a hundred.

When the sampling pump is operated intermittently this can occur during determined time periods with a speed which gives a flow proportional to the flow in the main conduit upstream of the branching point and downstream of the dialyzer.

If instead the sampling pump is operated intermittently with a constant speed this can occur during time periods which are proportional to the flow in the main conduit upstream of the branching point and downstream of the dialyzer.

If the amount of said substance in the extracted fraction is measured instead of its concentration, the volume of said fraction is also measured for calculating the concentration.

On determining the total amount of a substance extracted from, or supplied to, the patient, which substance, e.g. sodium, potassium, sodium bi-carbonate is already present in the fluid supplied to the dialyzer, the amount and/or proportion of the same substance supplied to the dialyzer is also calculated with the help of known and/or entered dialysis parameters.

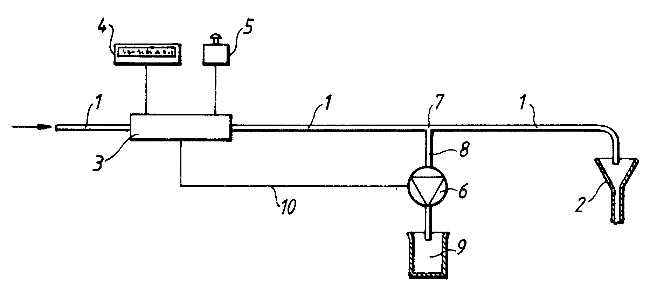

### DESCRIPTION OF THE DRAWING

The accompanying drawing shows a simple sketch of the principal of a system which can be used for carrying out the method according to the invention.

### SYSTEM DESCRIPTION

On the shown sketch reference numeral 1 denotes a conduit which is intended to lead the used dialysis fluid from a dialyzer (not shown) to a drain 2. A flow meter 3 is arranged in the conduit 1. With the help of this meter the total flow can be measured. Conveniently this can also be used for measuring the ultrafiltrate by comparing the total flow with the flow to the dialyzer. A display showing the obtained measured values is denoted by 4. 5 denotes a start and stop arrangement which can be manual or automatic, e.g. depending on a blood detector arranged just upstream of the dialyzer. A sampling pump is denoted by 6, said pump being arranged to supply a fraction of the flow in the main conduit 1 from a branching point 7 via a pre-branching conduit 8 to a sample collection vessel 9. A dashed line is denoted by 10, said dashed line being intended to show that the flow meter 3 can be arranged to control the pump 6 in order to give a determined proportional relationship between the flow in the main conduit 1 and the branch conduit 8.

The invention is of course not limited only to the example described above, but can be varied within the scope of the following claims. For example, the above described details can be arranged and combined with other measurement and control arrangements in a conventional dialysis monitor.

It should be noted that said fraction can also be extracted prior to the measurement of the total flow but that this must than be corrected with the extracted flow.

It should be further noted that certain monitors for haemofiltration mix the extracted filtrate with non-used replacement fluid so that a large amount of this mixture is obtained from the monitor. The invention can be used to advantage especially in this sort of process.

The expression "the total amount of used dialysis fluid" as used in the appended claims relates both to normal dialysis fluid as well as to the filtrate mixture or the pure filtrate which leaves the dialyzer (haemofilter) during haemodiafiltration or haemofiltration respectively.

## Claims

1. Method of measuring the effect of a dialysis or similar treatment, whereby a fraction of the used dialysis fluid is extraced downstream of the dialyzer for analysis of at least one substance, e.g. urea, whereby said fraction of the dialysis fluid is extracted via a branch conduit (8) containing a pump (6), **characterized** in that the total amount of used dialysis fluid is measured, in that the proportion and/or amount of said substance in the extracted fraction is measured and also in that the total amount of said substance removed from the patient is calculated with the help of these values.

2. Method according to claim 1, **characterized** in that said pump (6) is driven at a speed which produces a flow proportional to the flow in the main conduit (1) upstream of the branching point (7) and downstream of the dialyzer.

3. Method according to any preceding claim, **characterized** in that said pump (6) is continuously operated.

4. Method according to any preceding claim, **characterized** in that the flow in the branch conduit (8) is selected to be equal to one twentieth to on twenty-thousandth of the flow upstream of the branching point (7) and downstream of the dialyzer, suitably between one two-hundredth and one ten-thousandth and preferably about one five-thousandth of said flow.

5. Method according to any preceding claim, **characterized** in that the proportion of any of the following substances are measured in the extracted sample: urea, creatine , phosphate and beta-micro-globulin.

6. Method according to any preceding claim, **characterized** in that the sampling (6) is started automatically or manually, when it can be established that the first blood is approaching or has reached the dialyzer used, whereby there occurs a simultaneous start of the measurement of the total used amount of dialysis fluid, inclusive of the extracted ultrafiltrate.

7. Method according to claim 1, **characterized** in that the pump (6) is operated intermittently, whereby the number of sampling occurrences is selected to be at least ten, suitably over fifty and preferably considerably higher e.g. over a hundred.

8. Method according to claim 7, **characterized** in that the sampling pump (6) is operated for determined time periods with a speed which gives a flow proportional to the flow in the main conduit (1) upstream of the branching point (7) and downstream of the dialyzer.

9. Method according to claim 7, **characterized** in that the sampling pump (6) is operated at constant speed for time periods which are proportional to the flow in the main conduit (1) upstream of the branching point (7) and downstream of the dialyzer.

10. Method according to claim 1, **characterized** in that on measuring the amount of said substance in the extracted fraction, the volume of said fraction is also measured for calculating the concentration.

11. Method according to claim 1, **characterized** in that on determining the total amount of a substance extracted from or supplied to, to the patient, which substance, e.g. sodium, potassium or sodium bi-carbonate is already present in the fluid supplied to the dialyzer, the amount and/or proportion of the same substance supplied to the dialyzer is also calculated with the help of known and/or entered dialysis parameters.
